# EUROPEAN PATENT SPECIFICATION

(11) **EP 4 694 169 B1**
(45) Date of publication and mention of the grant of the patent: **04.11.2026**
(21) Application number: 25785580.9
(22) Date of filing: 19.03.2025
(51) Int. Cl.: H04N 23/70, A61B 1/00, A61B 1/045, A61B 1/06

(54) **DYNAMIC LUMINANCE CALIBRATION (DLC) METHOD**
VERFAHREN ZUR DYNAMISCHEN LUMINANZKALIBRIERUNG (DLC)
PROCÉDÉ D'ÉTALONNAGE DE LUMINANCE DYNAMIQUE (DLC)

(30) Priority: 13.06.2024 CN 202410756959
(43) Date of publication of application: 11.02.2026
(73) Proprietor: Weyo Surgical Technology Ltd., Nanjing, Jiangsu 210028 (CN)
(72) Inventor: REN, Zhiqiang, Nanjing, Jiangsu 210028 (CN); ZOU, Peng, Nanjing, Jiangsu 210028 (CN); SUN, Mingjian, Nanjing, Jiangsu 210028 (CN)
(74) Representative: Gille Hrabal Partnerschaftsgesellschaft mbB Patentanwälte
(86) International application number: PCT/CN2025/083297
(87) International publication number: WO 2025/214101

(56) References cited:
- CN-A- 110 099 222
- CN-A- 110 830 731
- CN-A- 112 911 146
- CN-A- 114 666 512
- CN-A- 118 338 131
- JP-A- 2004 283 421
- US-A1- 2023 255 443

## Description

### FIELD OF THE INVENTION

The present invention relates to the technical field of endoscope imaging, and in particular to a dynamic luminance calibration (D.L.C.) method.

### DESCRIPTION OF RELATED ART

In the field of endoscopic imaging, very high requirements are placed on the luminance stability of pictures. On the one hand, a stable picture can reduce the doctor's visual fatigue, and on the other hand, a stable picture can allow the doctor to face the surgical scene more calmly.

For image sensors, image luminance (LUMA) is determined by the exposure time (ET) and the analog gain amplification factor (GAIN). The working principle of the image sensor determines that the exposure time (ET) and the analog gain amplification factor (GAIN) are basically linearly related to the image luminance, so the image luminance can be expressed as $LUMA = k ∗ GAIN ∗ ET$

Therefore, the auto exposure algorithm needs to obtain the luminance of the current frame image and calculate the exposure time and analog gain magnification factor required for the next frame. For example, document US 2023/255443 A1 discloses a dynamic luminance calibration method comprising steps for auto metering on a dynamic video and performing auto dimming followed by a dynamic luminance adjustment.

Due to the existence of narrow and long channels in medical endoscope application scenarios, dark regions may still exist in pictures upon adjustment via the auto exposure algorithm, resulting in suboptimal luminance representation in dark regions of the pictures, thereby causing several typical issues as follows:
(1) The dimming is unstable, which causes non-linear luminance changes between frames and picture flickering during dimming.
(2) The dimming speed is slow, which results in long-term overexposure or underexposure of the pictures.
(3) Since the actual exposure register values are discrete, the dimming amplitude may be inaccurate, which results in that the picture luminance is unstable during the dimming, the auto exposure fails to reach optimal luminance, causing intermittent luminance fluctuations
(4) Overly bright proximal views and excessively dark distal views are presented in the narrow and long lumens.

### SUMMARY OF THE INVENTION

In view of this, an objective of the present invention is to propose a dynamic luminance calibration (D.L.C.) method to solve the existing problem that due to the existence of narrow and long channels in medical endoscope application scenarios, dark regions may still exist in pictures upon adjustment via the auto exposure algorithm, resulting in suboptimal luminance representation in dark regions of the pictures.

Based on the above objective, the present invention provides a dynamic luminance calibration (D.L.C.) method according to claim 1 and comprising the following steps:
S1: performing auto metering on a dynamic video;
S2: performing auto dimming on the dynamic video subjected to metering, wherein the auto metering and the auto dimming fall within the scope of auto exposure; and
S3: performing dynamic luminance adjustment on the video subjected to dimming, so that the picture luminance of the video is always in a reasonable state;
Step S1 comprises image valid region detection and auto metering, wherein the image valid region detection specifically comprises the following substeps:
   S1.1: selecting valid pixels in an image using a threshold parameter TH_valid; and
   S1.2: for each pixel (x, y) in an image I, if *I_{c}*(*x, y*)>TH valid, considering that the pixel (x, y) is a valid pixel, wherein *c* represents RGB triple channels, and a ratio of a number of valid pixels to a total number of pixels is denoted as ratio, wherein a TH valid value is set to 8 by default;
   and the auto metering specifically comprises the following substeps:
      S1.3: dividing the image into a plurality of blocks, with a total number of the blocks denoted as total_block, and calculating an average luminance Lᵢ of each block i sized as s * s, wherein a formula of the average luminance Lᵢ is as follows: ${L}_{i} \left(x, y\right) = \left⌊0.2126∗{R}_{i}\left(x, y\right)+0.7152∗{G}_{i}\left(x, y\right)+0.0722∗{B}_{i}\left(x, y\right)\right⌋$ ${L}_{i} = \frac{1}{{s}^{2}} {\sum }_{x = 1}^{s} {\sum }_{y = 1}^{s} {L}_{i} \left(x, y\right)$
      wherein, Lᵢ(x, y) indicates a luminance of a pixel in the block, Rᵢ(x, y), Gᵢ(x, y), Bᵢ(x, y) indicates values of the pixel (x, y) in the RGB triple channels, respectively, and | | indicates a floor operation;
      S1.4: calculating a number of selected blocks; $block = ratio ∗ total_block ;$ and
      S1.5: sorting the average luminances of all blocks in descending order, so that a metering result is; ${Y}_{L} = \left⌊\frac{S}{block}{\sum }_{j = 1}^{block} {L}_{j}\right⌋ + \left⌊\frac{1 - S}{{total}_{block} - block - 1}{\sum }_{block + 1}^{total_block} {L}_{j}\right⌋$
      wherein Lⱼ indicates the average luminance of the selected j-th block, and S is a tolerance parameter which can be set to any value between 0 and 1.

For an N-th frame image in a video stream, input conditions for an auto dimming module are: a current image luminance Y_{N} , a previous-frame image luminance Y_{N-1}, a target image luminance TY, exposure times ET_{N} and ET_{N-1}, analog gain amplification factors GAIN_{N} and GAIN_{N-1}; and a process of determining whether a dimming is required in S2 is as follows:
S2.1: when abs(TY - Y_{N}) < range, i.e., a current picture luminance is slightly different from a target luminance, maintaining exposure parameters unchanged, and exiting the auto dimming, wherein abs() represents an absolute value function, and range represents a threshold constant; and
S2.2: when abs(TY - Y_{N}) > range, entering a dimming process.

Preferably, the dimming process in step S2.2 comprises correcting an exposure model and calculating the exposure parameters, and the correcting the exposure model specifically comprises the following substeps:
S2.21: according to a working principle of an image sensor, under the condition of unchanged scenes, providing the following relationship: ${GAIN}_{N + 1} - {GAIN}_{N} = {K}_{1} ∗ \left({Y}_{N + 1}-{Y}_{N}\right)$ ${ET}_{N + 1} - {ET}_{N} = {K}_{2} ∗ \left({Y}_{N + 1}-{Y}_{N}\right) ;$
S2.22: setting K to an initial value which needs to be corrected in time for changing scenes, and in the dimming process, when one of GAIN and ET changes, recalculating a K value of a current frame based on a variation of the luminance, and when both change at the same time, expressing the K value as follows: ${K}_{1} = {K}_{1} ∗ \sqrt[2]{\frac{{\left({Y}_{N + 1}-{Y}_{N}\right)}^{2}}{\left({GAIN}_{N + 1}-{GAIN}_{N}\right) ∗ \left({ET}_{N + 1}-{ET}_{N}\right)}}$ ${K}_{2} = {K}_{2} ∗ \sqrt[2]{\frac{{\left({Y}_{N + 1}-{Y}_{N}\right)}^{2}}{\left({GAIN}_{N + 1}-{GAIN}_{N}\right) ∗ \left({ET}_{N + 1}-{ET}_{N}\right)}} ;$
S2.23: in order to improve the robustness of auto exposure during endoscope movement, taking the K value as a weighted value of the current frame and the previous frames, and after an estimated K value is obtained, estimating a variation of the exposure parameters based on a difference between the current frame luminance and the target luminance.

Preferably, the calculating the exposure parameters specifically comprises the following substeps:
S2.24: introducing n damping coefficients p to further adjust an intermediate process of dimming while a dimming speed can also be adjusted through the damping coefficients, wherein the damping coefficients p are expressed as follows: $p = \left\{\begin{matrix}{p}_{1} , \\ ⋮ \\ {p}_{i} , \\ ⋮ \\ {p}_{n} ,\end{matrix} \begin{matrix}th \leq {th}_{1} \\ ⋮ \\ {th}_{i - 1} < th \leq {th}_{i} \\ ⋮ \\ {th}_{n - 1} < th \leq {th}_{n}\end{matrix}\right.$ $th = \left⌊\frac{abs \left(TY-{Y}_{N}\right)}{{Y}_{N}}\right⌋ ;$
S2.25: since increasing the exposure time helps improve the signal-to-noise ratio, giving priority to using the exposure time during dimming, and adjusting the analog gain amplification factor only when the exposure time is insufficient to meet the luminance requirement, when GAIN = 1, disenabling GAIN, and adjusting ET: $ΔET = \left⌊p∗{K}_{2}∗\left(TY-{Y}_{N}\right)\right⌋$ ${ET}_{N + 1} = {ET}_{N} + ΔET$
calculating and issuing an actual register value Reg_{ET} to complete the current-frame dimming.

Preferably, when GAIN ≠ 1, GAIN is adjusted: $ΔGAIN = \left⌊p∗{K}_{1}∗\left(TY-{Y}_{N}\right)\right⌋$ ${GAIN}_{N + 1} = {GAIN}_{N} + ΔGAIN$
the actual register value Reg_{GAIN} is calculated at this time, and when ΔGAIN is less than a threshold th_g, it is further corrected through D*_{gain}*;
a difference between a magnification factor corresponding to the actual register value and an actual magnification factor of the previous frame is denoted as A, then: $A ∗ abs \left({D}_{gain}-1\right) = p ∗ {K}_{1} ∗ \left(TY-{Y}_{N}\right)$
wherein D*_{gain}* > 0, the adjusted D*_{gain}* can be calculated based on the relational expression, and finally, Reg_{ET} and Reg_{GAIN} are calculated for ending the dimming, and an N+1 frame image is obtained by multiplying by D*_{gain}* on the N+1 frame image.

According to the present invention, in step S3, the following substeps are included:
S3.1: dynamically determining whether to adjust a picture;
S3.2: performing adaptive dark region brightening; and
S3.3: performing dynamical contrast enhancement.

Preferably, the dynamically determining whether to adjust a picture in step S3.1 specifically comprises the following substeps:
S3.11: first, converting the image from an RGB domain to a YUV domain using a formula as follows: $Y = 0.1826 ∗ R + 0.6142 ∗ G + 0.062 ∗ B + 16$ $U = - 0.1006 ∗ R - 0.3386 ∗ G + 0.4392 ∗ B + 128$ $V = 0.4392 ∗ B - 0.3989 ∗ G - 0.0403 ∗ B + 128 ; and$
S3.12: performing histogram statistics on the Y channel, wherein during the histogram statistics,the idea of "image valid region detection in step S1" continues to be used, that is, only pixels with a luminance greater than TH valid are counted; after a luminance histogram Hist L is obtained, the numbers of pixels across all grayscale levels in Hist L are summed to obtain Hist L sum, then the number of pixels across each grayscale level in Hist L is divided by Hist L sum to obtain a weight for each grayscale level, as expressed in the following formula: $Hist _ L _ {w}_{j} = Hist _ {L}_{j} / Hist _ L _ sum ;$
S3.13: performing weighted summation on the weight and each grayscale value to obtain a weighted grayscale value of the image, as expressed in the following formula: $gray _ avg = {\sum }_{j = 0}^{255} Hist _ L _ {w}_{j} ∗ j$
in the histogram Hist_L, the number of pixels with grayscale levels less than TH_GT is counted as left_num and the number of pixels with grayscale levels greater than or equal to TH_GT is counted as right_num, wherein TH_GT defaults to 90, and *r*_*l*_*ratio* is a ratio of the number of pixels on the right side of TH_GT to the number of pixels on the left side of TH_GT, as expressed in the following formula: $r _ l _ ratio = right _ num / left _ num ;$ and
S3.14: when *gray*_*avg* is greater than or equal to TH_GT, considering that the picture is normal and requires no changes;
when *r_l_ratio* is greater than or equal to 1, considering that the picture is normal and requires no changes; except for the above two cases, considering that the picture is generally dark and requires dynamic brightening.

The adaptive dark region brightening in step S3.2 specifically comprises the following substeps:
S3.21: when the *gray*_*avg* value is small, considering that the picture has a low overall luminance and requires more enhancement for a dark region, in which case a bimodal distribution may appear such that it is necessary to introduce a *r_l_ratio* ratio, and the closer the ratio approaches 1, the higher the overall differentiation of the picture, indicating that the luminance/darkness distribution is appropriate, and there is no need to brighten the dark region.
S3.22: designing a curve dynamically adjusted based on *gray*_*avg* and *r_l_ratio* with a formula as follows: $f \left(x\right) = \left({x}^{b}∗{c}^{d}\right) / \left({x}^{b}∗{c}^{d}+{\left(1-x\right)}^{d}∗{a}^{b}\right)$
wherein x is a grayscale value ranging from 0 to 255, *f*(*x*) indicates that an original grayscale value will be mapped to a new grayscale value, wherein a is 0.3, and d is 1;
c is dynamically adjusted based on *gray*_*avg,* with a formula as follows: $c _ i = min \left(\left(TH_GT-gray_avg\right)/\left(TH_GT-40\right),1\right)$ $c = 0.3 + c _ i ∗ 0.3 ;$
b is dynamically adjusted based on *r_l_ratio* and *gray*_*avg*, wherein min represents a smaller one of the two values, with a formula as follows: $new _ r _ l _ ratio = \frac{\left(r_l_{ratio}^{3}∗{0.35}^{3}\right)}{\left(r_l_{ratio}^{3}∗{0.35}^{3}+{\left(1-r_l_ratio\right)}^{3}∗{0.5}^{3}\right)}$ $b _ i = 1 - c _ i ∗ \left(1-new_r_l_ratio\right)$ $b = min \left(0.35+0.65∗b_i,1\right) ;$ and
S3.23: applying a grayscale value of a new *f*(*x*) to the Y channel of the YUV image to obtain a new brightened YUV_new image.

Preferably, the dynamic contrast enhancement in stepS3.3 comprises the following substeps:
S3.31: first, converting YUV_new to RGB_new, i.e., converting the image from the YUV domain to the RGB domain;
S3.32: enhancing the RGB_new using an adaptive linear contrast method, with a specific enhancement formula as follows: $res _ {p}_{m}^{n} = clip \left(\left({p}_{m}^{n}-TH_GT\right)∗c_f+TH_GT,0,255\right)$
wherein, m in ${p}_{m}^{n}$ represents a pixel at a certain position, and n represents a certain color channel in RGB, *c_f* is a contrast enhancement factor, and a *c_f* formula is as follows: $c _ f = 1.4 - 0.4 ∗ b _ i$
wherein, a clip operation means that when a calculation result is less than 0, it will be truncated to 0, and when it is greater than 255, it will be truncated to 255, wherein $res _ {p}_{m}^{n}$ is a final graphical result.

Preferably, in step S3.31, a formula for converting the image from the YUV domain to the RGB domain is as follows: $R = 1.1644 ∗ \left(Y-16\right) + 1.7928 ∗ \left(V-128\right)$ $G = 1.1644 ∗ \left(Y-16\right) - 0.2133 ∗ \left(U-127\right) - 0.533 ∗ \left(V-128\right)$ $B = 1.1644 ∗ \left(Y-16\right) + 2.1124 ∗ \left(U-128\right) .$

The present invention has the following advantageous effects:
I. The valid region detection is added, which improves the adaptability of auto exposure in different endoscopy scenarios, and makes the auto metering results more stable.
II. The dimming process can correct the exposure theoretical model in real time, thereby ensuring the accuracy of the dimming model and improving the robustness of auto exposure during endoscope movement.
III. The damping coefficient is introduced into the dimming process to ensure the stability and speed of dimming. Since increasing the exposure time helps to improve the signal-to-noise ratio, the exposure time should be used first in the dimming process, and the analog gain amplification factor is adjusted when the exposure time is insufficient to meet the luminance requirements, thereby improving the accuracy of the dimming.
IV. For the scene of narrow and long lumens in endoscopic images, a dynamic luminance adjustment algorithm is designed, so that when the picture is dark, the luminance of the dark region of the picture can be adaptively adjusted, and when the luminance is dynamically adjusted, the adaptive contrast method is used to ensure that the picture still maintains a high contrast.
V. In summary, this method enables rapid and effective adjustment of exposure and picture luminance. During exposure adjustment, the image luminance quickly converges to the vicinity of the target luminance, the convergence process is smooth with comfortable visual perception, and the luminance remains stable after the completion of convergence without breathing effect. When the picture is dark, the luminance can be dynamically enhanced, and during dynamic luminance adjustment, the picture shall maintain sufficient contrast, so that the picture luminance is always in a reasonable state.

### BRIEF DESCRIPTION OF THE DRAWINGS

To describe the technical solutions in the embodiments of the present invention or in the prior art more clearly, the accompanying drawings required for describing the embodiments or the prior art are briefly introduced below. It is obvious that the accompanying drawings in the following description only show embodiments of the present invention, and other drawings may be obtained based on these drawings by those of ordinary skill in the art without creative efforts.
FIG. 1 is an overall flowchart according to an embodiment of the present invention;
FIG. 2 is an auto exposure flowchart according to an embodiment of the present invention;
FIG. 3 is an auto dimming flowchart according to an embodiment of the present invention;
FIG. 4 is a dynamic luminance adjustment flowchart according to an embodiment of the present invention;
FIG. 5 is an original image without dynamic luminance adjustment according to an embodiment of the present invention;
FIG. 6 is a result diagram illustrating the application of adaptive dark region brightening according to an embodiment of the present invention; and
FIG. 7 is a result diagram illustrating the combined application of adaptive dark region brightening and dynamic contrast enhancement according to an embodiment of the present invention.

### DETAILED DESCRIPTION OF THE EMBODIMENTS

To make the objectives, technical solutions and advantages of the present invention clearer, the present invention is further described in detail below with reference to specific embodiments and accompanying drawings.

It should be noted that, unless defined otherwise, technical or scientific terms used in the embodiments of the present invention shall have the same meanings as commonly understood by one of ordinary skill in the art to which the present invention belongs. The terms "first", "second", and similar expressions used in the present invention do not denote any order, quantity, or importance, but rather are used to distinguish different components. The terms "comprise", "include" or similar expressions mean that the elements or items preceding the terms cover the elements or items listed after the terms and their equivalents, without excluding other elements or items. The terms "connect", "couple" or similar expressions are not limited to physical or mechanical connections, but may include electrical connections, whether direct or indirect. The terms "upper", "lower", "left", "right" or similar expressions are only used to indicate relative positional relationships. When the absolute positions of described objects change, the relative positional relationships may change accordingly.

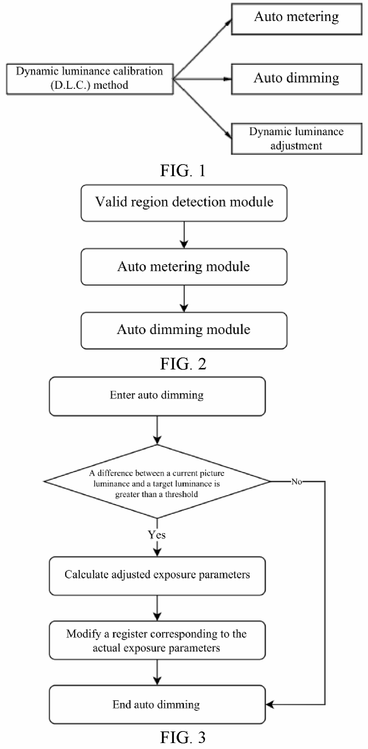

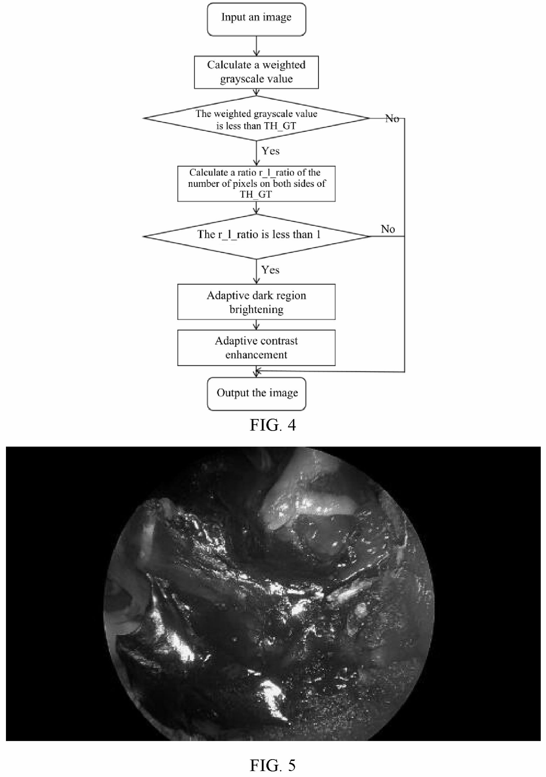

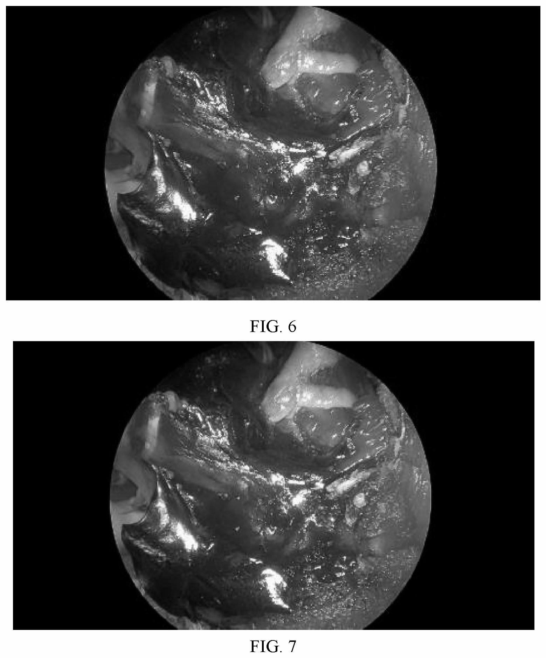

As shown in FIG. 1, FIG. 2, FIG. 3, FIG. 4, FIG. 5, FIG. 6 and FIG. 7, a dynamic luminance calibration (D.L.C.) method includes the following steps:
S1: Auto metering is performed on a dynamic video.

Step S1 includes image valid region detection and auto metering, wherein the image valid region detection specifically includes the following substeps:
S1.1: Valid pixels in an image are selected using a threshold parameter TH_valid.
S1.2: For each pixel (x, y) in an image I, if *I_{c}*(*x, y*)>TH_valid, it is considered that the pixel (x, y) is a valid pixel, where *c* represents RGB triple channels, and a ratio of a number of valid pixels to a total number of pixels is denoted as ratio, where a TH_valid value is set to 8 by default.

The auto metering specifically includes the following substeps:
S1.3: The image is divided into a plurality of blocks, where the size of each block may be 8*8, 16* 16 or 32*32 and a total number of the blocks is denoted as total_block; and an average luminance Lᵢ of each block i sized as s * s is calculated, where a formula of the average luminance Lᵢ is as follows: ${L}_{i} \left(x, y\right) = \left⌊0.2126∗{R}_{i}\left(x, y\right)+0.7152∗{G}_{i}\left(x, y\right)+0.0722∗{B}_{i}\left(x, y\right)\right⌋$ ${L}_{i} = \frac{1}{{s}^{2}} {\sum }_{x = 1}^{s} {\sum }_{y = 1}^{s} {L}_{i} \left(x, y\right)$
where, Lᵢ(x, y) indicates a luminance of a pixel in the block, Rᵢ(x, y), Gᵢ(x, y), Bᵢ(x, y) indicates values of the pixel (x, y) in the RGB triple channels, respectively, and └ ┘ indicates a floor operation; and this calculation method has been proven to be highly robust for bleeding scenarios.
S1.4: A number of selected blocks is calculated; $block = ratio ∗ total_block .$
S1.5: The average luminances of all blocks are sorted in descending order, so that a metering result is; ${Y}_{L} = \left⌊\frac{S}{block}{\sum }_{j = 1}^{block} {L}_{j}\right⌋ + \left⌊\frac{1 - S}{{total}_{block} - block - 1}{\sum }_{block + 1}^{total_block} {L}_{j}\right⌋$
where Lⱼ indicates the average luminance of the selected j-th block, and S is a tolerance parameter which can be set to any value between 0 and 1.
S2: Auto dimming is performed on the dynamic video subjected to metering, where the auto metering and the auto dimming fall within the scope of auto exposure.

For an N-th frame image in a video stream, input conditions for an auto dimming module are: a current image luminance Y_{N}, a previous-frame image luminance Y_{N-1}, a target image luminance TY, exposure times ET_{N} and ET_{N-1} , analog gain amplification factors GAIN_{N} and GAIN_{N-1}; and a process of determining whether a dimming is required in S2 is as follows:
S2.1: When abs(TY - Y_{N}) < range, i.e., a current picture luminance is slightly different from a target luminance, exposure parameters are maintained unchanged, and the auto dimming is exited, wherein abs() represents an absolute value function, and range represents a threshold constant.
S2.2: When abs(TY - Y_{N}) > range, a dimming process enters.

The dimming process in step S2.2 includes correcting an exposure model and calculating the exposure parameters, and the correcting the exposure model specifically includes the following substeps:
S2.21: According to a working principle of an image sensor, under the condition of unchanged scenes, the following relationship is provided: ${GAIN}_{N + 1} - {GAIN}_{N} = {K}_{1} ∗ \left({Y}_{N + 1}-{Y}_{N}\right)$ ${ET}_{N + 1} - {ET}_{N} = {K}_{2} ∗ \left({Y}_{N + 1}-{Y}_{N}\right) .$
S2.22: K is set to an initial value which needs to be corrected in time for changing scenes, and in the dimming process, when one of GAIN and ET changes, a K value of a current frame is recalculated based on a variation of the luminance, and when both change at the same time, the K value is expressed as follows: ${K}_{1} = {K}_{1} ∗ \sqrt[2]{\frac{{\left({Y}_{N + 1}-{Y}_{N}\right)}^{2}}{\left({GAIN}_{N + 1}-{GAIN}_{N}\right) ∗ \left({ET}_{N + 1}-{ET}_{N}\right)}}$ ${K}_{2} = {K}_{2} ∗ \sqrt[2]{\frac{{\left({Y}_{N + 1}-{Y}_{N}\right)}^{2}}{\left({GAIN}_{N + 1}-{GAIN}_{N}\right) ∗ \left({ET}_{N + 1}-{ET}_{N}\right)}} .$
S2.23: In order to improve the robustness of auto exposure during endoscope movement, the K value may be a weighted value of the current frame and the previous frames, and after an estimated K value is obtained, a variation of the exposure parameters may be estimated based on a difference between the current frame luminance and the target luminance.

The calculating the exposure parameters specifically includes the following substeps:
Some thresholds should be set in the dimming process to limit the adjustment range of exposure parameters to avoid excessive or insufficient adjustments. For example, the maximum ΔET/ET_{N} and the maximum ΔGAIN/GAIN_{N} are set.

S2.24: n damping coefficients p are introduced to further adjust an intermediate process of dimming while a dimming speed can also be adjusted through the damping coefficients, where the damping coefficients p are expressed as follows: $p = \left\{\begin{matrix}{p}_{1} , \\ ⋮ \\ {p}_{i} , \\ ⋮ \\ {p}_{n} ,\end{matrix} \begin{matrix}th \leq {th}_{1} \\ ⋮ \\ {th}_{i - 1} < th \leq {th}_{i} \\ ⋮ \\ {th}_{n - 1} < th \leq {th}_{n}\end{matrix}\right.$ $th = \left⌊\frac{abs \left(TY-{Y}_{N}\right)}{{Y}_{N}}\right⌋ .$

S2.25: Since increasing the exposure time helps improve the signal-to-noise ratio, the exposure time should be used first during dimming, and the analog gain amplification factor is adjusted again when the exposure time is insufficient to meet the luminance requirement, when GAIN = 1, GAIN is disenabled, and ET is adjusted: $ΔET = \left⌊p∗{K}_{2}∗\left(TY-{Y}_{N}\right)\right⌋$ ${ET}_{N + 1} = {ET}_{N} + ΔET$

An actual register value Reg_{ET} is calculated and issued to complete the current-frame dimming.

When GAIN ≠ 1, GAIN is adjusted: $ΔGAIN = \left⌊p∗{K}_{1}∗\left(TY-{Y}_{N}\right)\right⌋$ ${GAIN}_{N + 1} = {GAIN}_{N} + ΔGAIN$

The actual register value Reg_{GAIN} is calculated at this time, and when ΔGAIN is less than a threshold th_g, it is further corrected through D*_{gain}.*

A difference between a magnification factor corresponding to the actual register value and an actual magnification factor of the previous frame is denoted as A, then: $A ∗ abs \left({D}_{gain}-1\right) = p ∗ {K}_{1} ∗ \left(TY-{Y}_{N}\right)$ where D*_{gain}* > 0, the adjusted D*_{gain}* can be calculated based on the relational expression, and finally, Reg_{ET} and Reg_{GAIN} are calculated for ending the dimming, and an N+1 frame image is obtained by multiplying by D*_{gain}* on the N+1 frame image.

S3: Dynamic luminance adjustment is performed on the video subjected to dimming, so that the picture luminance of the video is always in a reasonable state.

In step S3, the following substeps are included:
S3.1: Whether to adjust a picture is dynamically determined.

The dynamically determining whether to adjust a picture in step S3.1 specifically includes the following substeps:
S3.11: First, the image is converted from an RGB domain to a YUV domain using a formula as follows: $Y = 0.1826 ∗ R + 0.6142 ∗ G + 0.062 ∗ B + 16$ $U = - 0.1006 ∗ R - 0.3386 ∗ G + 0.4392 ∗ B + 128$ $V = 0.4392 ∗ B - 0.3989 ∗ G - 0.0403 ∗ B + 128 .$
S3.12: Histogram statistics is performed on the Y channel, where during the histogram statistics,the idea of "image valid region detection in step S1" continues to be used, that is, only pixels with a luminance greater than TH_valid are counted; after a luminance histogram Hist_L is obtained, the numbers of pixels across all grayscale levels in Hist_L are summed to obtain Hist_L_sum, then the number of pixels across each grayscale level in Hist_L is divided by Hist_L_sum to obtain a weight for each grayscale level, as expressed in the following formula: $Hist _ L _ {w}_{j} = Hist _ {L}_{j} / Hist _ L _ sum .$
S3.13: Weighted summation is performed on the weight and each grayscale value to obtain a weighted grayscale value of the image, as expressed in the following formula: $gray _ avg = {\sum }_{j = 0}^{255} Hist _ L _ {w}_{j} ∗ j$

In the histogram Hist_L, the number of pixels with grayscale levels less than TH_GT is counted as left_num and the number of pixels with grayscale levels greater than or equal to TH_GT is counted as right_num, where TH_GT defaults to 90, and *r*_*l*_*ratio* is a ratio of the number of pixels on the right side of TH_GT to the number of pixels on the left side of TH_GT, as expressed in the following formula: $r _ l _ ratio = right _ num / left _ num .$

S3.14: When *gray*_*avg* is greater than or equal to TH_GT, it is considered that the picture is normal and requires no changes; and
when *r*_*l*_*ratio* is greater than or equal to 1, it is considered that the picture is normal and requires no changes; except for the above two cases, it is considered that that the picture is generally dark and requires dynamic brightening.

S3.2: Adaptive dark region brightening is performed.

The adaptive dark region brightening in step S3.2 specifically includes the following substeps:
S3.21: When the *gray*_*avg* value is small, it is considered that the picture has a low overall luminance and requres more enhancement for a dark region, in which case a bimodal distribution may appear such that it is necessary to introduce a *r*_*l*_*ratio* ratio, and the closer the ratio approaches 1, the higher the overall differentiation of the picture, indicating that the luminance/darkness distribution is appropriate, and there is no need to brighten the dark region.

S3.22: A curve dynamically adjusted based on *gray*_*avg* and *r*_*l*_*ratio* is designed, with a formula as follows: $f \left(x\right) = \left({x}^{b}∗{c}^{d}\right) / \left({x}^{b}∗{c}^{d}+{\left(1-x\right)}^{d}∗{a}^{b}\right)$ where x is a grayscale value ranging from 0 to 255, *f*(*x*) indicates that an original grayscale value will be mapped to a new grayscale value, where a is 0.3, and d is 1.

c is dynamically adjusted based on *gray*_*avg,* with a formula as follows: $c _ i = min \left(\left(TH_GT-gray_avg\right)/\left(TH_GT-40\right),1\right)$ $c = 0.3 + c _ i ∗ 0.3 .$

b is dynamically adjusted based on *r*_*l*_*ratio* and *gray*_*avg,* wherein min represents a smaller one of the two values, with a formula as follows: $new _ r _ l _ ratio = \frac{\left(r_l_{ratio}^{3}∗{0.35}^{3}\right)}{\left(r_l_{ratio}^{3}∗{0.35}^{3}+{\left(1-r_l_ratio\right)}^{3}∗{0.5}^{3}\right)}$ $b _ i = 1 - c _ i ∗ \left(1-new_r_l_ratio\right)$ $b = min \left(0.35+0.65∗b_i,1\right) .$

S3.23: A grayscale value of a new *f*(*x*) is applied to the Y channel of the YUV image to obtain a new brightened YUV_new image.

S3.3: Dynamical contrast enhancement is performed.

When the enhancement curve is applied for certain enhancement, the picture contrast is generally reduced. At this time, a contrast enhancement method is required to restore partial contrast, thereby improving the visual perception of the picture.

The dynamic contrast enhancement in step S3.3 specifically includes the following substeps:
S3.31: First, YUV_new is converted to RGB_new, i.e., the image is converted from the YUV domain to the RGB domain.

In step S3.31, a formula for converting the image from the YUV domain to the RGB domain is as follows: $R = 1.1644 ∗ \left(Y-16\right) + 1.7928 ∗ \left(V-128\right)$ $G = 1.1644 ∗ \left(Y-16\right) - 0.2133 ∗ \left(U-127\right) - 0.533 ∗ \left(V-128\right)$ $B = 1.1644 ∗ \left(Y-16\right) + 2.1124 ∗ \left(U-128\right) .$

S3.32: The RGB_new is enhanced using an adaptive linear contrast method, with a specific enhancement formula as follows: $res _ {p}_{m}^{n} = clip \left(\left({p}_{m}^{n}-TH_GT\right)∗c_f+TH_GT,0,255\right)$ where, m in ${p}_{m}^{n}$ represents a pixel at a certain position, and n represents a certain color channel in RGB, *c_f* is a contrast enhancement factor, and a *c*_*f* formula is as follows: $c _ f = 1.4 - 0.4 ∗ b _ i$ where, a clip operation means that when a calculation result is less than 0, it will be truncated to 0, and when it is greater than 255, it will be truncated to 255, wherein $res _ {p}_{m}^{n}$ is a final graphical result.

Those skilled in the art should understand that the discussion of any of the above embodiments is merely illustrative and is not intended to imply that the scope of the present invention is limited to these examples. Under the concept of the present invention, the technical features in the above embodiments or different embodiments may be combined, the steps may be implemented in any order, and there are many other variations of the different aspects of the present invention as described above, which are not provided in detail for the sake of simplicity.

The embodiments of the present invention are intended to cover all such substitutions, modifications and variations that fall within the broad scope of the appended claims.

## Claims

1. A dynamic luminance calibration method, **characterized by** comprising the following steps:
S1: performing auto metering on a dynamic video;
S2: performing auto dimming on the dynamic video subjected to metering, wherein the auto metering and the auto dimming fall within the scope of auto exposure; and
S3: performing dynamic luminance adjustment on the video subjected to dimming, so that the picture luminance of the video is always in a reasonable state;
Step S1 comprises image valid region detection and auto metering, wherein the image valid region detection specifically comprises the following substeps:
S1.1: selecting valid pixels in an image using a threshold parameter TH_valid; and
S1.2: for each pixel (x,y) in an image I, if *I_{c}*(*x, y*)>TH valid, considering that the pixel (x, y) is a valid pixel, wherein *c* represents RGB triple channels, and a ratio of a number of valid pixels to a total number of pixels is denoted as ratio, wherein a TH valid value is set to 8 by default;
and the auto metering specifically comprises the following substeps:
S1.3: dividing the image into a plurality of blocks, with a total number of the blocks denoted as total_block, and calculating an average luminance Lᵢ of each block i sized as s * s, wherein a formula of the average luminance Lᵢ is as follows: ${L}_{i} \left(x, y\right) = \left⌊0.2126∗{R}_{i}\left(x, y\right)+0.7152∗{G}_{i}\left(x, y\right)+0.0722∗{B}_{i}\left(x, y\right)\right⌋$ ${L}_{i} = \frac{1}{{s}^{2}} {\sum }_{x = 1}^{s} {\sum }_{y = 1}^{s} {L}_{i} \left(x, y\right)$
wherein, Lᵢ(x, y) indicates a luminance of a pixel in the block, Rᵢ(x, y), Gᵢ(x, y), Bᵢ(x, y) indicates values of the pixel (x, y) in the RGB triple channels, respectively, and | | indicates a floor operation;
S1.4: calculating a number of selected blocks; $block = ratio ∗ total_block ;$ and
S1.5: sorting the average luminances of all blocks in descending order, so that a metering result is; ${Y}_{L} = \left|\frac{S}{block}{\sum }_{j = 1}^{block} {L}_{j}\right| + \left|\frac{1 - S}{{total}_{block} - block - 1}{\sum }_{block + 1}^{total_block} {L}_{j}\right|$
wherein Lⱼ indicates the average luminance of the selected j-th block, and S is a tolerance parameter which can be set to any value between 0 and 1;
for an N-th frame image in a video stream, input conditions for an auto dimming module are: a current image luminance Y_{N}, a previous-frame image luminance Y_{N-1}, a target image luminance TY, exposure times ET_{N} and ET_{N-1} , analog gain amplification factors GAIN_{N} and GAIN_{N-1}; and a process of determining whether a dimming is required in S2 is as follows:
S2.1: when abs(TY - Y_{N}) < range, i.e., a current picture luminance is slightly different from a target luminance, maintaining exposure parameters unchanged, and exiting the auto dimming, wherein abs() represents an absolute value function, and range represents a threshold constant; and
S2.2: when abs(TY - Y_{N}) > range, entering a dimming process;
in step S3, the following substeps are included:
S3.1: dynamically determining whether to adjust a picture;
S3.2: performing adaptive dark region brightening; and
S3.3: performing dynamical contrast enhancement;
the dynamically determining whether to adjust a picture in step S3.1 specifically comprises the following substeps:
S3.11: first, converting the image from an RGB domain to a YUV domain using a formula as follows: $Y = 0.1826 ∗ R + 0.6142 ∗ G + 0.062 ∗ B + 16$ $U = - 0.1006 ∗ R - 0.3386 ∗ G + 0.4392 ∗ B + 128$ $V = 0.4392 ∗ B - 0.3989 ∗ G - 0.0403 ∗ B + 128 ;$
S3.12: performing histogram statistics on the Y channel, wherein during the histogram statistics, only pixels with a luminance greater than TH_valid are counted, after a luminance histogram Hist_L is obtained, the numbers of pixels across all grayscale levels in Hist_L are summed to obtain Hist L_sum, then the number of pixels across each grayscale level in Hist_L is divided by Hist_L_sum to obtain a weight for each grayscale level, as expressed in the following formula: $Hist _ L _ {w}_{j} = Hist _ {L}_{j} / Hist _ L _ sum ;$
S3.13: performing weighted summation on the weight and each grayscale value to obtain a weighted grayscale value of the image, as expressed in the following formula: $gray _ avg = {\sum }_{j = 0}^{255} Hist _ L _ {w}_{j} ∗ j$
in the histogram Hist_L, the number of pixels with grayscale levels less than TH_GT is counted as left_num and the number of pixels with grayscale levels greater than or equal to TH_GT is counted as right_num, wherein TH_GT defaults to 90, and *r*_*l*_*ratio* is a ratio of the number of pixels on the right side of TH_GT to the number of pixels on the left side of TH_GT, as expressed in the following formula: $r _ l _ ratio = right _ num / left _ num ;$ and
S3.14: when *gray*_*avg* is greater than or equal to TH_GT, considering that the picture is normal and requires no changes;
when *r*_*l*_*ratio* is greater than or equal to 1, considering that the picture is normal and requires no changes; except for the above two cases, considering that the picture is generally dark and requires dynamic brightening;
the adaptive dark region brightening in step S3.2 specifically comprises the following substeps:
S3.21: when the *gray_avg* value is small, considering that the picture has a low overall luminance and requires more enhancement for a dark region, in which case a bimodal distribution may appear such that it is necessary to introduce a *r*_*l*_*ratio* ratio, and the closer the ratio approaches 1, the higher the overall differentiation of the picture, indicating that the luminance/darkness distribution is appropriate, and there is no need to brighten the dark region.
S3.22: designing a curve dynamically adjusted based on *gray*_*avg* and *r*_*l*_*ratio* with a formula as follows: $f \left(x\right) = \left({x}^{b}∗{c}^{d}\right) / \left({x}^{b}∗{c}^{d}+{\left(1-x\right)}^{d}∗{a}^{b}\right)$
wherein x is a grayscale value ranging from 0 to 255, *f*(*x*) indicates that an original grayscale value will be mapped to a new grayscale value, wherein a is 0.3, and d is 1;
c is dynamically adjusted based on *gray*_*avg,* with a formula as follows: $c _ i = min \left(\left(TH_GT-gray_avg\right)/\left(TH_GT-40\right),1\right)$ $c = 0.3 + c _ i ∗ 0.3 ;$
b is dynamically adjusted based on *r*_*l*_*ratio* and *gray*_*avg,* wherein min represents a smaller one of the two values, with a formula as follows: $new _ r _ l _ ratio = \frac{\left(r_l_{ratio}^{3}∗{0.35}^{3}\right)}{\left(r_l_{ratio}^{3}∗{0.35}^{3}+{\left(1-r_l_ratio\right)}^{3}∗{0.5}^{3}\right)}$ $b _ i = 1 - c _ i ∗ \left(1-new_r_l_ratio\right)$ $b = min \left(0.35+0.65∗b_i,1\right) ;$ and
S3.23: applying a grayscale value of a new *f*(*x*) to the Y channel of the YUV image to obtain a new brightened YUV_new image;
the dynamic contrast enhancement in step S3.3 comprises the following substeps:
S3.31: first, converting YUV new to RGB new, i.e., converting the image from the YUV domain to the RGB domain;
S3.32: enhancing the RGB new using an adaptive linear contrast method, with a specific enhancement formula as follows: $res _ {p}_{m}^{n} = clip \left(\left({p}_{m}^{n}-TH_GT\right)∗c_f+TH_GT,0,255\right)$
wherein, m in ${p}_{m}^{n}$ represents a pixel at a certain position, and n represents a certain color channel in RGB, *c_f* is a contrast enhancement factor, and a *c*_*f* formula is as follows: $c _ f = 1.4 - 0.4 ∗ b _ i$
wherein, a clip operation means that when a calculation result is less than 0, it will be truncated to 0, and when it is greater than 255, it will be truncated to 255, wherein $res _ {p}_{m}^{n}$ is a final graphical result.

2. The dynamic luminance calibration method according to claim 1, **characterized in that** the dimming process in step S2.2 comprises correcting an exposure model and calculating the exposure parameters,and the correcting the exposure model specifically comprises the following substeps:
S2.21: according to a working principle of an image sensor, under the condition of unchanged scenes, providing the following relationship: ${GAIN}_{N + 1} - {GAIN}_{N} = {K}_{1} ∗ \left({Y}_{N + 1}-{Y}_{N}\right)$ ${ET}_{N + 1} - {ET}_{N} = {K}_{2} ∗ \left({Y}_{N + 1}-{Y}_{N}\right) ;$
S2.22: setting K to an initial value which needs to be corrected in time for changing scenes, and in the dimming process, when one of GAIN and ET changes, recalculating a K value of a current frame based on a variation of the luminance, and when both change at the same time, expressing the K value as follows: ${K}_{1} = {K}_{1} ∗ \sqrt[2]{\frac{{\left({Y}_{N + 1}-{Y}_{N}\right)}^{2}}{\left({GAIN}_{N + 1}-{GAIN}_{N}\right) ∗ \left({ET}_{N + 1}-{ET}_{N}\right)}}$ ${K}_{2} = {K}_{2} ∗ \sqrt[2]{\frac{{\left({Y}_{N + 1}-{Y}_{N}\right)}^{2}}{\left({GAIN}_{N + 1}-{GAIN}_{N}\right) ∗ \left({ET}_{N + 1}-{ET}_{N}\right)}} ; and$
S2.23: in order to improve the robustness of auto exposure during endoscope movement, taking the K value as a weighted value of the current frame and the previous frames, and after an estimated K value is obtained, estimating a variation of the exposure parameters based on a difference between the current frame luminance and the target luminance.

3. The dynamic luminance calibration method according to claim 2, **characterized in that** the calculating exposure parameters specifically comprises the following substeps:
S2.24: introducing n damping coefficients p to further adjust an intermediate process of dimming while a dimming speed can also be adjusted through the damping coefficients, wherein the damping coefficients p are expressed as follows: $p = \left\{\begin{matrix}{p}_{1} , \\ ⋮ \\ {p}_{i} , \\ ⋮ \\ {p}_{n} ,\end{matrix} \begin{matrix}th \leq {th}_{1} \\ ⋮ \\ {th}_{i - 1} < th \leq {th}_{i} \\ ⋮ \\ {th}_{n - 1} < th \leq {th}_{n}\end{matrix}\right.$ $th = \left⌊\frac{abs \left(TY-{Y}_{N}\right)}{{Y}_{N}}\right⌋ ;$
S2.25: since increasing the exposure time helps improve the signal-to-noise ratio, giving priority to using the exposure time during dimming, and adjusting the analog gain amplification factor only when the exposure time is insufficient to meet the luminance requirement, when GAIN = 1, disenabling GAIN, and adjusting ET: $ΔET = \left⌊p∗{K}_{2}∗\left(TY-{Y}_{N}\right)\right⌋$ ${ET}_{N + 1} = {ET}_{N} + ΔET$
calculating and issuing an actual register value Reg_{ET} to complete the current-frame dimming.

4. The dynamic luminance calibration method according to claim 3, **characterized in that**, when GAIN ≠ 1, GAIN is adjusted: $ΔGAIN = \left⌊p∗{K}_{1}∗\left(TY-{Y}_{N}\right)\right⌋$ ${GAIN}_{N + 1} = {GAIN}_{N} + ΔGAIN$
the actual register value Reg_{GAIN} is calculated at this time, and when ΔGAIN is less than a threshold th_g, it is further corrected through D*_{gain}*;
a difference between a magnification factor corresponding to the actual register value and an actual magnification factor of the previous frame is denoted as A, then: $A ∗ abs \left({D}_{gain}-1\right) = p ∗ {K}_{1} ∗ \left(TY-{Y}_{N}\right)$
wherein D*_{gain}* > 0, the adjusted D*_{gain}* can be calculated based on the relational expression, and finally, Reg_{ET} and Reg_{GAIN} are calculated for ending the dimming, and an N+1 frame image is obtained by multiplying by D*_{gain}* on the N+1 frame image.

5. The dynamic luminance calibration method according to claim 1, **characterized in that**, in step S3.31, a formula for converting the image from the YUV domain to the RGB domain is as follows: $R = 1.1644 ∗ \left(Y-16\right) + 1.7928 ∗ \left(V-128\right)$ $G = 1.1644 ∗ \left(Y-16\right) - 0.2133 ∗ \left(U-127\right) - 0.533 ∗ \left(V-128\right)$ $B = 1.1644 ∗ \left(Y-16\right) + 2.1124 ∗ \left(U-128\right) .$

## Patentansprüche

1. Ein dynamisches Luminanzkalibrierungsverfahren, **dadurch gekennzeichnet, dass** es die folgenden Schritte umfasst:
S1: Durchführen einer automatischen Messung an einem dynamischen Video;
S2: Durchführen einer automatischen Dimmung an dem der Messung unterzogenen dynamischen Video, wobei die automatische Messung und die automatische Dimmung in den Bereich der automatischen Belichtung fallen; und
S3: Durchführen einer dynamischen Luminanzanpassung an dem der Dimmung unterzogenen Video, so dass die Bildluminanz des Videos stets in einem angemessenen Zustand ist;
wobei Schritt S1 eine Bildgültigkeitsbereichserkennung und eine automatische Messung umfasst, wobei die Bildgültigkeitsbereichserkennung spezifisch die folgenden Unterschritte umfasst:
S1.1: Auswählen gültiger Pixel in einem Bild unter Verwendung eines Schwellenwertparameters TH_valid; und
S1.2: für jedes Pixel (x,y) in einem Bild I, wenn *I_{c}*(*x, y*)>TH valid, Berücksichtigen, dass das Pixel (x, y) ein gültiges Pixel ist, wobei *c* RGB-Dreifachkanäle darstellt, und ein Verhältnis einer Anzahl gültiger Pixel zu einer Gesamtanzahl von Pixeln als *ratio* bezeichnet wird, wobei ein TH valid-Wert standardmäßig auf 8 gesetzt ist;
und wobei die automatische Messung spezifisch die folgenden Unterschritte umfasst:
S1.3: Unterteilen des Bildes in eine Vielzahl von Blöcken, wobei eine Gesamtanzahl der Blöcke als *total_block* bezeichnet wird, und Berechnen einer durchschnittlichen Luminanz *Lᵢ* jedes Blocks *i* mit einer Größe von *s* * *s*, wobei eine Formel der durchschnittlichen Luminanz *Lᵢ* wie folgt lautet: ${L}_{i} \left(x, y\right) = \left⌊0,2126∗{R}_{i}\left(x, y\right)+0,7152∗{G}_{i}\left(x, y\right)+0,0722∗{B}_{i}\left(x, y\right)\right⌋$ ${L}_{i} = \frac{1}{{s}^{2}} {\sum }_{x = 1}^{s} {\sum }_{y = 1}^{s} {L}_{i} \left(x, y\right)$
wobei *Lᵢ*(*x, y*) eine Luminanz eines Pixels in dem Block angibt, *Rᵢ*(*x, y*)*, Gᵢ*(*x, y*), *Bᵢ*(*x, y*) jeweils Werte des Pixels (*x, y*) in den RGB-Dreifachkanälen angibt, und └ ┘ eine Abrundungsoperation angibt;
S1.4: Berechnen einer Anzahl ausgewählter *blocks*; $block = ratio ∗ total _ block ;$ und
S1.5: Sortieren der durchschnittlichen Luminanzen aller Blöcke in absteigender Reihenfolge, so dass ein Messergebnis ist; ${Y}_{L} = \left⌊\frac{S}{block}{\sum }_{j = 1}^{block} {L}_{j}\right⌋ + \left⌊\frac{1 - S}{{total}_{block} - block - 1}{\sum }_{block + 1}^{total _ block} {L}_{j}\right⌋$
wobei *Lⱼ* die durchschnittliche Luminanz des ausgewählten j-ten Blocks angibt, und S ein Toleranzparameter ist, der auf einen beliebigen Wert zwischen 0 und 1 gesetzt werden kann;
wobei für ein N-tes Rahmenbild in einem Videostrom Eingangsbedingungen für ein automatisches Dimmungsmodul sind: eine aktuelle Bildluminanz *Y_{N},* eine Bildluminanz des vorherigen Rahmens *Y*_{*N*-1}, eine Zielbildluminanz *TY,* Belichtungszeiten *ET_{N}* und *ET*_{*N*-1}, analoge Verstärkungsfaktoren *GAIN_{N}* und *GAIN*_{*N*-1}; und wobei ein Prozess zum Bestimmen, ob eine Dimmung in S2 erforderlich ist, wie folgt ist:
S2.1: wenn *abs*(*TY - Y_{N}*) *< range,* d. h. eine aktuelle Bildluminanz sich geringfügig von einer Zielluminanz unterscheidet, Beibehalten der Belichtungsparameter unverändert und Beenden der automatischen Dimmung, wobei abs() eine Absolutwertfunktion darstellt und *range* eine Schwellenwertkonstante darstellt; und
S2.2: wenn *abs*(*TY - Y_{N}*) *> range,* Eintreten in einen Dimmungsprozess;
wobei in Schritt S3 die folgenden Unterschritte enthalten sind:
S3.1: dynamisches Bestimmen, ob ein Bild angepasst werden soll;
S3.2: Durchführen einer adaptiven Aufhellung dunkler Bereiche; und
S3.3: Durchführen einer dynamischen Kontrastverbesserung;
wobei das dynamische Bestimmen, ob ein Bild angepasst werden soll, in Schritt S3.1 spezifisch die folgenden Unterschritte umfasst:
S3.11: zunächst Umwandeln des Bildes von einem RGB-Bereich in einen YUV-Bereich unter Verwendung einer Formel wie folgt: $Y = 0 , 1826 ∗ R + 0 , 6142 ∗ G + 0 , 062 ∗ B + 16$ $U = - 0 , 1006 ∗ R - 0 , 3386 ∗ G + 0 , 4392 ∗ B + 128$ $V = 0 , 4392 ∗ B - 0 , 3989 ∗ G - 0 , 0403 ∗ B + 128 ;$
S3.12: Durchführen einer Histogrammstatistik am Y-Kanal, wobei während der Histogrammstatistik nur Pixel mit einer Luminanz größer als TH_valid gezählt werden, wobei nach Erhalt eines Luminanzhistogramms Hist_L die Anzahlen der Pixel über alle Graustufen in Hist_L summiert werden, um Hist_L_sum zu erhalten, wobei dann die Anzahl der Pixel über jede Graustufe in Hist_L durch Hist_L_sum geteilt wird, um ein Gewicht für jede Graustufe zu erhalten, wie in der folgenden Formel ausgedrückt: $Hist _ L _ {w}_{j} = Hist _ {L}_{j} / Hist _ L _ sum ;$
S3.13: Durchführen einer gewichteten Summierung des Gewichts und jedes Graustufenwerts, um einen gewichteten Graustufenwert des Bildes zu erhalten, wie in der folgenden Formel ausgedrückt: $gray _ avg = {\sum }_{j = 0}^{255} Hist _ L _ {w}_{j} ∗ j$
wobei im Histogramm Hist_L die Anzahl der Pixel mit Graustufen kleiner als TH_GT als left_num gezählt wird und die Anzahl der Pixel mit Graustufen größer oder gleich TH_GT als right_num gezählt wird, wobei TH_GT standardmäßig 90 ist, und *r*_*l*_*ratio* ein Verhältnis der Anzahl der Pixel auf der rechten Seite von TH_GT zur Anzahl der Pixel auf der linken Seite von TH_GT ist, wie in der folgenden Formel ausgedrückt: $r _ l _ ratio = right _ num / left _ num ;$ und
S3.14: wenn *gray*_*avg* größer oder gleich TH_GT ist, Berücksichtigen, dass das Bild normal ist und keine Änderungen erfordert;
wenn *r*_*l*_*ratio* größer oder gleich 1 ist, Berücksichtigen, dass das Bild normal ist und keine Änderungen erfordert; außer in den obigen zwei Fällen, Berücksichtigen, dass das Bild allgemein dunkel ist und eine dynamische Aufhellung erfordert;
wobei die adaptive Aufhellung dunkler Bereiche in Schritt S3.2 spezifisch die folgenden Unterschritte umfasst:
S3.21: wenn der *gray_avg*-Wert klein ist, Berücksichtigen, dass das Bild eine niedrige Gesamtluminanz hat und mehr Verbesserung für einen dunklen Bereich erfordert, wobei in diesem Fall eine bimodale Verteilung auftreten kann, so dass es notwendig ist, ein *r_l_ratio*-Verhältnis einzuführen, und je näher das Verhältnis an 1 herankommt, desto höher ist die Gesamtdifferenzierung des Bildes, was anzeigt, dass die Luminanz/Dunkelheitsverteilung angemessen ist, und es nicht notwendig ist, den dunklen Bereich aufzuhellen;
S3.22: Entwerfen einer Kurve, die dynamisch basierend auf *gray*_*avg* und *r*_*l*_*ratio* angepasst ist, mit einer Formel wie folgt: $f \left(x\right) = \left({x}^{b}∗{c}^{d}\right) / \left({x}^{b}∗{c}^{d}+{\left(1-x\right)}^{d}∗{a}^{b}\right)$
wobei x ein Graustufenwert im Bereich von 0 bis 255 ist, *f*(*x*) angibt, dass ein ursprünglicher Graustufenwert auf einen neuen Graustufenwert abgebildet wird, wobei a 0,3 ist und d 1 ist;
wobei c dynamisch basierend auf *gray*_*avg* angepasst wird, mit einer Formel wie folgt: $c _ i = min \left(\left(TH_GT-gray_avg\right)/\left(TH_GT-40\right),1\right)$ $c = 0 , 3 + c _ i ∗ 0 , 3 ;$
wobei b dynamisch basierend auf *r*_*l*_*ratio* und *gray*_*avg*angepasst wird, wobei min den kleineren der zwei Werte darstellt, mit einer Formel wie folgt: $new _ r _ l _ ratio = \frac{\left(r_l_{ratio}^{3}∗{0 , 35}^{3}\right)}{\left(r_l_{ratio}^{3}∗{0 , 35}^{3}+{\left(1-r_l_ratio\right)}^{3}∗{0 , 5}^{3}\right)}$ $b _ i = 1 - c _ i ∗ \left(1-new_r_l_ratio\right)$ $b = min \left(0,35+0,65∗b_i,1\right) ;$ und
S3.23: Anwenden eines Graustufenwerts eines neuen *f*(*x*) auf den Y-Kanal des YUV-Bildes, um ein neues aufgehelltes YUV_new-Bild zu erhalten;
wobei die dynamische Kontrastverbesserung in Schritt S3.3 die folgenden Unterschritte umfasst:
S3.31: zunächst Umwandeln von YUV new in RGB new, d. h. Umwandeln des Bildes vom YUV-Bereich in den RGB-Bereich;
S3.32: Verbessern von RGB new unter Verwendung eines adaptiven linearen Kontrastverfahrens, mit einer spezifischen Verbesserungsformel wie folgt: $res _ {p}_{m}^{n} = clip \left(\left({p}_{m}^{n}-TH_GT\right)∗c_f+TH_GT,0,255\right)$
wobei m in ${p}_{m}^{n}$ ein Pixel an einer bestimmten Position darstellt und n einen bestimmten Farbkanal in RGB darstellt, *c_f* ein Kontrastverbesserungsfaktor ist, und eine *c_f-*Formel wie folgt lautet: $c _ f = 1 , 4 - 0 , 4 ∗ b _ i$
wobei eine Clip-Operation bedeutet, dass wenn ein Berechnungsergebnis kleiner als 0 ist, es auf 0 abgeschnitten wird, und wenn es größer als 255 ist, es auf 255 abgeschnitten wird, wobei $res _ {p}_{m}^{n}$ ein endgültiges grafisches Ergebnis ist.

2. Das dynamische Luminanzkalibrierungsverfahren nach Anspruch 1, **dadurch gekennzeichnet, dass** der Dimmungsprozess in Schritt S2.2 ein Korrigieren eines Belichtungsmodells und ein Berechnen der Belichtungsparameter umfasst, und wobei das Korrigieren des Belichtungsmodells spezifisch die folgenden Unterschritte umfasst:
S2.21: gemäß einem Arbeitsprinzip eines Bildsensors, unter der Bedingung unveränderter Szenen, Bereitstellen der folgenden Beziehung: ${GAIN}_{N + 1} - {GAIN}_{N} = {K}_{1} ∗ \left({Y}_{N + 1}-{Y}_{N}\right)$ ${ET}_{N + 1} - {ET}_{N} = {K}_{2} ∗ \left({Y}_{N + 1}-{Y}_{N}\right) ;$
S2.22: Setzen von K auf einen Anfangswert, der für sich ändernde Szenen rechtzeitig korrigiert werden muss, und im Dimmungsprozess, wenn sich einer von *GAIN* und *ET* ändert, Neuberechnen eines K-Werts eines aktuellen Rahmens basierend auf einer Variation der Luminanz, und wenn sich beide gleichzeitig ändern, Ausdrücken des K-Werts wie folgt: ${K}_{1} = {K}_{1} ∗ \sqrt[2]{\frac{{\left({Y}_{N + 1}-{Y}_{N}\right)}^{2}}{\left({GAIN}_{N + 1}-{GAIN}_{N}\right) ∗ \left({ET}_{N + 1}-{ET}_{N}\right)}}$ ${K}_{2} = {K}_{2} ∗ \sqrt[2]{\frac{{\left({Y}_{N + 1}-{Y}_{N}\right)}^{2}}{\left({GAIN}_{N + 1}-{GAIN}_{N}\right) ∗ \left({ET}_{N + 1}-{ET}_{N}\right)}} ;$ und
S2.23: um die Robustheit der automatischen Belichtung während einer Endoskopbewegung zu verbessern, Nehmen des K-Werts als gewichteten Wert des aktuellen Rahmens und der vorherigen Rahmen, und nachdem ein geschätzter K-Wert erhalten wurde, Schätzen einer Variation der Belichtungsparameter basierend auf einer Differenz zwischen der aktuellen Rahmenluminanz und der Zielluminanz.

3. Das dynamische Luminanzkalibrierungsverfahren nach Anspruch 2, **dadurch gekennzeichnet, dass** das Berechnen der Belichtungsparameter spezifisch die folgenden Unterschritte umfasst:
S2.24: Einführen von n Dämpfungskoeffizienten p zur weiteren Anpassung eines Zwischenprozesses der Dimmung, während eine Dimmungsgeschwindigkeit auch durch die Dämpfungskoeffizienten angepasst werden kann, wobei die Dämpfungskoeffizienten p wie folgt ausgedrückt werden: $p = \left\{\begin{matrix}{p}_{1} , \\ ⋮ \\ {p}_{i} , \\ ⋮ \\ {p}_{n} ,\end{matrix} \begin{matrix}th \leq {th}_{1} \\ ⋮ \\ {th}_{i - 1} < th \leq {th}_{i} \\ ⋮ \\ {th}_{n - 1} < th \leq {th}_{n}\end{matrix}\right.$ $th = \left⌊\frac{abs \left(TY-{Y}_{N}\right)}{{Y}_{N}}\right⌋ ;$
S2.25: da das Erhöhen der Belichtungszeit hilft, das Signal-Rausch-Verhältnis zu verbessern, Priorisieren der Verwendung der Belichtungszeit während der Dimmung, und Anpassen des analogen Verstärkungsfaktors nur wenn die Belichtungszeit nicht ausreicht, um die Luminanzanforderung zu erfüllen, wenn *GAIN =* 1, Deaktivieren von *GAIN,* und Anpassen von *ET:* $Δ ET = \left⌊p∗{K}_{2}∗\left(TY-{Y}_{N}\right)\right⌋$ ${ET}_{N + 1} = {ET}_{N} + Δ ET$
Berechnen und Ausgeben eines tatsächlichen Registerwerts *Reg_{ET}* zum Abschließen der Dimmung des aktuellen Rahmens.

4. Das dynamische Luminanzkalibrierungsverfahren nach Anspruch 3, **dadurch gekennzeichnet, dass** wenn *GAIN* ≠ 1, *GAIN* angepasst wird: $Δ GAIN = \left⌊p∗{K}_{1}∗\left(TY-{Y}_{N}\right)\right⌋$ ${GAIN}_{N + 1} = {GAIN}_{N} + Δ GAIN$
wobei der tatsächliche Registerwert *Reg_{GAIN}* zu diesem Zeitpunkt berechnet wird, und wenn Δ*GAIN* kleiner als ein Schwellenwert th_g ist, er weiter durch *D_{gain}* korrigiert wird;
wobei eine Differenz zwischen einem Vergrößerungsfaktor, der dem tatsächlichen Registerwert entspricht, und einem tatsächlichen Vergrößerungsfaktor des vorherigen Rahmens als A bezeichnet wird, dann: $A ∗ abs \left({D}_{gain}-1\right) = p ∗ {K}_{1} ∗ \left(TY-{Y}_{N}\right)$
wobei *D_{gain} >* 0, das angepasste *D_{gain}* basierend auf dem Beziehungsausdruck berechnet werden kann, und schließlich *Reg_{ET}* und *Reg_{GAIN}* zum Beenden der Dimmung berechnet werden, und ein N+1-Rahmenbild durch Multiplizieren mit *D_{gain}* auf dem N+1-Rahmenbild erhalten wird.

5. Das dynamische Luminanzkalibrierungsverfahren nach Anspruch 1, **dadurch gekennzeichnet, dass** in Schritt S3.31 eine Formel zum Umwandeln des Bildes vom YUV-Bereich in den RGB-Bereich wie folgt lautet: $R = 1 , 1644 ∗ \left(Y-16\right) + 1 , 7928 ∗ \left(V-128\right)$ $G = 1 , 1644 ∗ \left(Y-16\right) - 0 , 2133 ∗ \left(U-127\right) - 0 , 533 ∗ \left(V-128\right)$ $B = 1 , 1644 ∗ \left(Y-16\right) + 2 , 1124 ∗ \left(U-128\right) .$

## Revendications

1. Procédé d'étalonnage dynamique de la luminance, **caractérisé en ce qu'**il comprend les étapes suivantes:
S1: effectuer une mesure automatique sur une vidéo dynamique;
S2: effectuer un assombrissement automatique de la vidéo dynamique ayant fait l'objet de la mesure, la mesure automatique et l'assombrissement automatique s'inscrivant dans le cadre de l'exposition automatique; et
S3: effectuer un ajustement dynamique de la luminance sur la vidéo ayant subi l'atténuation, de sorte que la luminance de l'image de la vidéo soit toujours dans un état approprié;
l'étape S1 comprenant une détection de la zone valide de l'image et une mesure automatique, la détection de la zone valide de l'image comprenant plus précisément les sous-étapes suivantes:
S1.1: sélectionner les pixels valides dans une image à l'aide d'un paramètre de seuil TH_valid; et
S1.2: pour chaque pixel (x, y) d'une image I, si *I_{c}*(*x, y)* > TH_valid, considérer que le pixel (x, y) est un pixel valide, où c représente les trois canaux RVB, et où le rapport entre le nombre de pixels valides et le nombre total de pixels est désigné par ratio, la valeur de TH_valid étant fixée par défaut à 8;
et la mesure automatique comprenant spécifiquement les sous-étapes suivantes:
S1.3: diviser l'image en une pluralité de blocs, le nombre total de blocs étant désigné par total_block, et calculer une luminance moyenne *Lᵢ* de chaque bloc i de taille *s* * *s*, la formule de la luminance moyenne *Lᵢ* étant la suivante: ${L}_{i} \left(x, y\right) = \left⌊0,2126∗{R}_{i}\left(x, y\right)+0,7152∗{G}_{i}\left(x, y\right)+0,0722∗{B}_{i}\left(x, y\right)\right⌋$ ${L}_{i} = \frac{1}{{s}^{2}} {\sum }_{x = 1}^{s} {\sum }_{y = 1}^{s} {L}_{i} \left(x, y\right)$
où *Lᵢ*(*x, y*) désigne la luminance d'un pixel dans le bloc, *Rᵢ*(*x, y*), *Gᵢ*(*x, y*), *Bᵢ*(*x, y*) désigne respectivement les valeurs du pixel(*x*, *y*) dans les trois canaux RVB, et └ ┘ désigne une opération d'arrondi;
S1.4: calculer un nombre de blocks sélectionnés; $block = ratio ∗ total _ block ;$ et
S1.5: trier les luminances moyennes de tous les blocs par ordre décroissant, de manière à obtenir un résultat de mesure; ${Y}_{L} = \left⌊\frac{S}{block}{\sum }_{j = 1}^{block} {L}_{j}\right⌋ + \left⌊\frac{1 - S}{{total}_{block} - block - 1}{\sum }_{block + 1}^{total _ block} {L}_{j}\right⌋$
où *Lⱼ* désigne la luminance moyenne du j-ième bloc sélectionné, et S est un paramètre de tolérance pouvant être défini sur une valeur quelconque comprise entre 0 et 1;
où, pour une N-ième image d'une séquence vidéo, les conditions d'entrée d'un module de gradation automatique sont: une luminance d'image actuelle *Y_{N},* une luminance d'image de l'image précédente *Y*_{*N*-1}, une luminance d'image cible *TY,* des temps d'exposition *ET_{N}* et *E T*_{*N*-1}, des facteurs d'amplification analogiques *GAIN_{N}* et *GAIN*_{*N*-1}; et où un processus permettant de déterminer si une gradation est nécessaire en S2 est le suivant:
S2.1: si *abs*(*TY - Y_{N}*) *< range,* c'est-à-dire si la luminance actuelle de l'image diffère légèrement d'une luminance cible, maintenir les paramètres d'exposition inchangés et mettre fin à la gradation automatique, où abs() représente une fonction de valeur absolue et *range* représente une constante de seuil; et
S2.2: si *abs*(*TY - Y_{N}*) *> range,* lancer un processus de gradation;
l'étape S3 comprenant les sous-étapes suivantes:
S3.1: déterminer de manière dynamique si une image doit être ajustée;
S3.2: effectuer un éclaircissement adaptatif des zones sombres; et
S3.3: effectuer une amélioration dynamique du contraste;
la détermination dynamique à l'étape S3.1 de la nécessité d'ajuster une image comprenant spécifiquement les sous-étapes suivantes:
S3.11: convertir d'abord l'image d'un espace RVB en un espace YUV à l'aide d'une formule telle que: $Y = 0 , 1826 ∗ R + 0 , 6142 ∗ G + 0 , 062 ∗ B + 16$ $U = - 0 , 1006 ∗ R - 0 , 3386 ∗ G + 0 , 4392 ∗ B + 128$ $V = 0 , 4392 ∗ B - 0 , 3989 ∗ G - 0 , 0403 ∗ B + 128 ;$
S3.12: effectuer une analyse statistique de l'histogramme sur le canal Y, en ne comptant, lors de cette analyse, que les pixels dont la luminance est supérieure à TH_valid, après obtention d'un histogramme de luminance Hist_L, les nombres de pixels pour tous les niveaux de gris dans Hist_L sont additionnés pour obtenir Hist_L_sum, puis le nombre de pixels pour chaque niveau de gris dans Hist_L est divisé par Hist_L_sum afin d'obtenir un poids pour chaque niveau de gris, comme l'exprime la formule suivante: $Hist _ L _ {w}_{j} = Hist _ {L}_{j} / Hist _ L _ sum ;$
S3.13: effectuer une sommation pondérée du poids et de chaque valeur de niveau de gris afin d'obtenir une valeur de niveau de gris pondérée de l'image, telle qu'exprimée par la formule suivante: $gray _ avg = {\sum }_{j = 0}^{255} Hist _ L _ {w}_{j} ∗ j$
où, dans l'histogramme Hist_L, le nombre de pixels présentant des niveaux de gris inférieurs à TH_GT est compté comme left_num et le nombre de pixels présentant des niveaux de gris supérieurs ou égaux à TH_GT est compté comme right_num, TH_GT étant par défaut égal à 90, et où *r_l_ratio* est un rapport entre le nombre de pixels situés à droite de TH_GT et le nombre de pixels situés à gauche de TH_GT, tel qu'exprimé par la formule suivante: $r _ l _ ratio = right _ num / left _ num ;$ et
S3.14: si *gray*_*avg* est supérieur ou égal à TH_GT, considérer que l'image est normale et ne nécessite aucune modification;
si *r_l_ratio* est supérieur ou égal à 1, considérer que l'image est normale et ne nécessite aucune modification; sauf dans les deux cas ci-dessus, considérer que l'image est globalement sombre et nécessite un éclaircissement dynamique;
l'éclaircissement adaptatif des zones sombres à l'étape S3.2 comprenant spécifiquement les sous-étapes suivantes:
S3.21: si la valeur de *gray*_*avg* est faible, considérer que l'image présente une faible luminance globale et nécessite davantage d'amélioration au niveau des zones sombres, un cas où une distribution bimodale peut se produire, rendant nécessaire l'introduction d'un rapport *r_l_ratio,* et plus ce rapport se rapproche de 1, plus la différenciation globale de l'image est élevée, ce qui indique que la répartition de la luminance et de l'obscurité est adéquate et qu'il n'est pas nécessaire d'éclaircir les zones sombres;
S3.22: conception d'une courbe ajustée dynamiquement en fonction de *gray*_*avg* et *r_l_ratio,* à l'aide d'une formule telle que: $f \left(x\right) = \left({x}^{b}∗{c}^{d}\right) / \left({x}^{b}∗{c}^{d}+{\left(1-x\right)}^{d}∗{a}^{b}\right)$
où x est une valeur de niveau de gris comprise entre 0 et 255, *f*(*x*) indique qu'une valeur de niveau de gris d'origine est mappée sur une nouvelle valeur de niveau de gris, a étant égal à 0,3 et d à 1;
où c est ajusté de manière dynamique en fonction de *gray_arg,* selon la formule suivante: $c _ i = min \left(\left(TH_GT-gray_avg\right)/\left(TH_GT-40\right),1\right)$ $c = 0 , 3 + c _ i ∗ 0 , 3 ;$
où b est ajusté de manière dynamique en fonction de *r_l_ratio* et de *gray*_*avg,* min représentant la plus petite des deux valeurs, selon la formule suivante: $new _ r _ l _ ratio = \frac{\left(r_l_{ratio}^{3}∗{0 , 35}^{3}\right)}{\left(r_l_{ratio}^{3}∗{0 , 35}^{3}+{\left(1-r_l_ratio\right)}^{3}∗{0 , 5}^{3}\right)}$ $b _ i = 1 - c _ i ∗ \left(1-new_r_l_ratio\right)$ $b = min \left(0,35+0,65∗b_i,1\right) ;$ et
S3.23: application d'une valeur en niveaux de gris d'un nouvel *f*(*x*) au canal Y de l'image YUV afin d'obtenir une nouvelle image éclaircie YUV_new;
l'amélioration dynamique du contraste à l'étape S3.3 comprenant les sous-étapes suivantes:
S3.31: convertir d'abord YUV new en RGB new, c'est-à-dire convertir l'image de l'espace YUV vers l'espace RGB;
S3.32: améliorer RGB new à l'aide d'un procédé de contraste linéaire adaptatif, avec une formule d'amélioration spécifique comme suit: $res _ {p}_{m}^{n} = clip \left(\left({p}_{m}^{n}-TH_GT\right)∗c_f+TH_GT,0,255\right)$
où m dans ${p}_{m}^{n}$ représente un pixel à une position donnée et n représente un canal de couleur donné dans RGB, *c_f* est un facteur d'amélioration du contraste, et la formule *c*_*f* s'écrit comme suit: $c _ f = 1 , 4 - 0 , 4 ∗ b _ i$
où une opération de clip signifie que si un résultat de calcul est inférieur à 0, il est écrêté à 0, et s'il est supérieur à 255, il est écrêté à 255, $res _ {p}_{m}^{n}$ étant un résultat graphique final.

2. Procédé d'étalonnage dynamique de la luminance selon la revendication 1, **caractérisé en ce que** le processus de gradation de l'étape S2.2 comprend une correction d'un modèle d'exposition et un calcul des paramètres d'exposition, et dans lequel la correction du modèle d'exposition comprend spécifiquement les sous-étapes suivantes:
S2.21: conformément au principe de fonctionnement d'un capteur d'image, à condition que la scène reste inchangée, établir la relation suivante: ${GAIN}_{N + 1} - {GAIN}_{N} = {K}_{1} ∗ \left({Y}_{N + 1}-{Y}_{N}\right)$ ${ET}_{N + 1} - {ET}_{N} = {K}_{2} ∗ \left({Y}_{N + 1}-{Y}_{N}\right) ;$
S2.22: définir K sur une valeur initiale qui doit être corrigée en temps utile en cas de changement de scène, et, au cours du processus de gradation, lorsqu'une des valeurs suivantes change: *GAIN* ou *ET,* recalculer une valeur K d'une image actuelle en fonction d'une variation de la luminance, et lorsque les deux changent simultanément, exprimer la valeur K comme suit: ${K}_{1} = {K}_{1} ∗ \sqrt[2]{\frac{{\left({Y}_{N + 1}-{Y}_{N}\right)}^{2}}{\left({GAIN}_{N + 1}-{GAIN}_{N}\right) ∗ \left({ET}_{N + 1}-{ET}_{N}\right)}}$ ${K}_{2} = {K}_{2} ∗ \sqrt[2]{\frac{{\left({Y}_{N + 1}-{Y}_{N}\right)}^{2}}{\left({GAIN}_{N + 1}-{GAIN}_{N}\right) ∗ \left({ET}_{N + 1}-{ET}_{N}\right)}} ;$
S2.23: afin d'améliorer la robustesse de l'exposition automatique pendant un mouvement de l'endoscope, prendre la valeur K comme valeur pondérée de l'image actuelle et des images précédentes, et après avoir obtenu une valeur K estimée, estimer une variation des paramètres d'exposition sur la base d'une différence entre la luminance de l'image actuelle et la luminance cible.

3. Procédé d'étalonnage dynamique de la luminance selon la revendication 2, **caractérisé en ce que** le calcul des paramètres d'exposition comprend spécifiquement les sous-étapes suivantes:
S2.24: introduction de n coefficients d'atténuation p pour ajuster davantage un processus intermédiaire de gradation, tandis qu'une vitesse de gradation peut également être ajustée par les coefficients d'atténuation, les coefficients d'atténuationp étant exprimés comme suit: $p = \left\{\begin{matrix}{p}_{1} , \\ ⋮ \\ {p}_{i} , \\ ⋮ \\ {p}_{n} ,\end{matrix} \begin{matrix}th \leq {th}_{1} \\ ⋮ \\ {th}_{i - 1} < th \leq {th}_{i} \\ ⋮ \\ {th}_{n - 1} < th \leq {th}_{n}\end{matrix}\right.$ $th = \left⌊\frac{abs \left(TY-{Y}_{N}\right)}{{Y}_{N}}\right⌋ ;$
S2.25: étant donné que l'augmentation du temps d'exposition contribue à améliorer le rapport signal/bruit, privilégier l'utilisation du temps d'exposition pendant la gradation, et ajuster le facteur d'amplification analogique uniquement lorsque le temps d'exposition est insuffisant pour satisfaire l'exigence de luminance, lorsque *GAIN =* 1, désactiver *GAIN,* et ajuster *ET:* $Δ ET = \left⌊p∗{K}_{2}∗\left(TY-{Y}_{N}\right)\right⌋$ ${ET}_{N + 1} = {ET}_{N} + Δ ET$
calculer et fournir une valeur de registre réelle Reg_{ET} pour achever la gradation de l'image en cours.

4. Procédé d'étalonnage dynamique de la luminance selon la revendication 3, **caractérisé en ce que**, lorsque *GAIN* ≠ 1 et *GAIN* sont ajustés: $Δ GAIN = \left⌊p∗{K}_{1}∗\left(TY-{Y}_{N}\right)\right⌋$ ${GAIN}_{N + 1} = {GAIN}_{N} + Δ GAIN$
la valeur de registre réelle *Reg_{GAIN}* étant calculée à ce moment-là, et si ΔGAIN est inférieure à une valeur seuil th_g, elle est ensuite corrigée par D*_{gain}*;
où une différence entre un facteur d'agrandissement correspondant à la valeur réelle du registre et un facteur d'agrandissement réel de l'image précédent est désignée par A, alors: $A ∗ abs \left({D}_{gain}-1\right) = p ∗ {K}_{1} ∗ \left(TY-{Y}_{N}\right)$
où *D_{gain} >* 0, la valeur ajustée de *D_{gain}* pouvant être calculée à partir de l'expression de relation, et enfin Reg_{ET} et *Reg_{GAIN}* sont calculés pour mettre fin à l'atténuation, et une image de trame N+1 est obtenue en multipliant par *D_{gain}* sur l'image de trame N+1.

5. Procédé d'étalonnage dynamique de la luminance selon la revendication 1, **caractérisé en ce que**, à l'étape S3.31, une formule permettant de convertir l'image de l'espace YUV vers l'espace RVB est la suivante: $R = 1 , 1644 ∗ \left(Y-16\right) + 1 , 7928 ∗ \left(V-128\right)$ $G = 1 , 1644 ∗ \left(Y-16\right) - 0 , 2133 ∗ \left(U-127\right) - 0 , 533 ∗ \left(V-128\right)$ $B = 1 , 1644 ∗ \left(Y-16\right) + 2 , 1124 ∗ \left(U-128\right) .$
